# EUROPÄISCHE PATENTSCHRIFT

(11) **EP 1 976 619 B1**
(45) Veröffentlichungstag und Bekanntmachung des Hinweises auf die Patenterteilung: **08.02.2012**
(21) Anmeldenummer: 06841288.1
(22) Anmeldetag: 07.12.2006
(51) Int. Cl.: B01D 61/02, C08F 2/38, C07C 41/06, C07C 67/04

(54) **VERFAHREN ZUR ABTRENNUNG VON METALL-KOMPLEXKATALYSATOREN AUS TELOMERISATIONSGEMISCHEN**
METHOD FOR THE ELIMINATION OF METAL COMPLEX CATALYSTS FROM TELOMERIZATION MIXTURES
PROCÉDÉ DE SÉPARATION DE CATALYSEURS RENFERMANT UN COMPLEXE MÉTALLIQUE À PARTIR DE MÉLANGES DE TÉLOMÉRISATION

(30) Priorität: 26.01.2006 DE 102006003618
(43) Veröffentlichungstag der Anmeldung: 08.10.2008
(73) Patentinhaber: Evonik Oxeno GmbH, 45772 Marl (DE)
(72) Erfinder: BAUMGARTEN, Goetz, 45721 Haltern am See (DE); ORTMANN, Dagmara, CH-3902 Brig-Glis (CH); KUPPINGER, Franz-Felix, 45768 Marl (DE); BORGMANN, Cornelia, 60486 Frankfurt (DE); HOUBRECHTS, Stephan, B-2570 Duffel (BE)
(86) Internationale Anmeldenummer: PCT/EP2006/069401
(87) Internationale Veröffentlichungsnummer: WO 2007/085321

(56) Entgegenhaltungen:
- EP-A- 1 103 303
- WO-A-03/053572
- WO-A-2006/024616
- US-A1- 5 243 099
- US-A1- 5 302 750

## Beschreibung

Die vorliegende Erfindung betrifft ein Verfahren zur Abtrennung von Metallkomplexkatalysatoren aus Reaktionsgemischen, die bei der Telomerisation erhalten werden.

Die Telomerisation von nicht cyclischen Olefinen mit mindestens zwei konjugierten Doppelbindungen, insbesondere die Herstellung von 1-Octa-2,7-dienylderivaten durch Umsetzung eines 1,3-Butadien-haltigen Kohlenwasserstoffgemischs, insbesondere Crack-C₄, mit Nucleophilen, ist eine in jüngster Zeit häufig beschriebene und untersuchte Reaktion.

Die dabei aus zwei Molen 1,3-Butadien und einem Mol Nucleophil entstehenden Telomerisationsprodukte (ungesättigte Amine, ungesättigte Alkohole und deren ungesättigte Ester und ungesättigte Ether) sind Ausgangsstoffe für organische Synthesen. Die sauerstoffhaltigen Derivate sind Vorstufen für die Herstellung von linearen C₈-Alkoholen und C₈-Olefinen, insbesondere 1-Octanol und 1-Octen. 1-Octanol wiederum wird beispielsweise zur Erzeugung von Weichmachern verwendet. 1-Octen ist ein begehrtes Comonomer zur Modifizierung von Polyethylen und Polypropylen.

Die Telomerisation von Butadien mit einem Nucleophil zu Octadienylderivaten wird durch Metallkomplexe, insbesondere Palladiumverbindungen, katalysiert.

Beispiele für Telomerisationsreaktionen werden unter anderem beschrieben in E. J. Smutny, J. Am. Chem. Soc. 1967, 89, 6793; S. Takahashi, T. Shibano, N. Hagihara, Tetrahedron Lett. 1967, 2451; EP 0 561 779, US 3,499,042, US 3,530,187, GB 1 178 812, GB 1 248 593, US 3,670,029, US 3,670,032, US 3,769,352, US 3,887,627, GB 1 354 507, DE 20 40 708, US 4,142,060, US 4,146,738, US 4,196,135, GB 1 535 718, US 4,104,471, DE 21 61 750 und EP 0 218 100.

In DE 195 23 335 erfolgt die Abtrennung des Katalysatorsystems durch Umsetzung des Katalysatorsystems mit einem wasserlöslichen Liganden und anschließende Extraktion mit einer Hexan/Wassermischung, bei der das Telomerisierungsprodukt in der Hexanphase und der Katalysator in der wässrigen Phase erhalten wird.

In EP 0 561 779 wird die Abtrennung des Katalysators durch Destillation, Fällung oder Extraktion beschrieben.

In DE 101 49 348 wird die Abtrennung des Katalysators aus dem Telomerisierungsgemisch durch Destillation, Extraktion, Fällung oder Adsorption abgetrennt und gegebenenfalls ganz oder teilweise in die Telomerisationsreaktion zurückgefahren.

In DE 103 29 042 wird bezüglich der Telomerisierung auf die vorgenannten Schutzrechte Bezug genommen.

In DE 103 08 111 wird allgemein das Abtrennen von gelöst oder kolloidal vorliegenden Feststoffen, insbesondere Katalysatoren aus Lösungen in nicht wässrigen Lösemittel mit Hilfe einer Membran beschrieben.

US5243099A1 offenbart einen Prozess zur Telomerisation von konjugierten Diolefinen mit einem Palladiumkomplex-Katalysator mit Organophosphat-Liganden im Verhältnis 5:1. Der Katalysator wird aus dem Reaktionsgemisch mittels einer stets druckbetriebenen Ultrafiltration abgetrennt.

US5302750A1 offenbart einen Prozess zur Herstellung von n-Oktandienolen aus Butadienen mittels Telomerisation mit einem Palladiumkomplex-Katalysator mit Organophosphat-Liganden in der Gegenwart von basischen tertiären Aminen, wobei der Katalysator mittels Membrantechnologie zurückgewonnen und rezykliert wird. Hierbei werden beispielsweise hydrophobe PVDF-Membranen eingesetzt.

EP 1103303A offenbart die Verwendung von Membranen zur Abtrennung von Rhodium-Organophosphor-Ligand-Komplexkatalysatoren aus einem Reaktionsgemisch enthaltend u.a. Olefine. Als Membranwerkstoffe werden Keramik oder Polydimethylsiloxan mit einer Trenngrenze (MWCO) von etwa 700 Da genannt. Die Druckdifferenz beträgt 1 bis 3.9 MPa. Die Differenz im Löslichkeitsprodukt 0 bis 400 √(kJ/m³). Das Verfahren kann mehrstufig sein.

Nachteilig an den aus dem Stand der Technik bekannten Verfahren ist der häufig auftretende Verlust von Katalysator, insbesondere des Katalysatormetalls bei der Abtrennung des Katalysatorsystems aus der Reaktionsmischung. Bei der thermischen Abtrennung des Katalysatorsystems tritt häufig eine Zersetzung des Metallkomplexkatalysators auf und das Metall des Komplexkatalysators scheidet an den Wänden der verwendeten Apparate ab. Eine Rückgewinnung des Metalls ist oft nur unter hohem Aufwand möglich. Insbesondere wenn das Katalysatormetall ein teures Edelmetall ist, leidet die Wirtschaftlichkeit von Telomerisationsverfahren unter dem Verlust an teuerem Katalysatormetall bei der Katalysatorabtrennung. Ebenfalls häufig zu beobachten ist, dass durch das Abtrennverfahren, z. B. durch die thermische Abtrennung, die Fällung oder die Extraktion die Aktivität des abgetrennten Katalysatorsystems abnimmt, so dass dem rückgeführten Katalysatorsystem große Mengen an Frischkatalysator zugeführt werden muss oder weitere Aufarbeitungsschritte notwendig sind, um die gewünschte Aktivität zu erreichen. Zudem sind einige der im Stand der Technik vorgeschlagenen Verfahren, insbesondere die Extraktionsverfahren sehr aufwändig.

Ausgehend von diesem Stand der Technik bestand die Aufgabe der vorliegenden Erfindung darin, ein Verfahren bereitzustellen, welches einen oder mehrere der Nachteile der Verfahren des Standes der Technik nicht aufweist. Insbesondere bestand die Aufgabe der vorliegenden Erfindung darin, ein einfaches Abtrennverfahren bereitzustellen, bei welchem vorzugsweise der Metall-Komplexkatalysator möglichst vollständig und mit einem möglichst geringen Aktivitätsverlust abgetrennt werden kann.

Nachteilig an den aus dem Stand der Technik bekannten Verfahren ist weiterhin, dass die meisten der bekannten lösemittelstabilen Membranen nicht alkalistabil sind bzw. alkalistabile Membranen keine ausreichende Lösemittelstabilität aufweisen. Weiterhin sollte eine Membran bereitgestellt werden, die unter den Reaktionsbedingungen stabil nicht nur gegen das Lösemittelsystem sondern auch gegen die stark alkalischen Reaktionsbedingungen ist.

Überraschenderweise wurde gefunden, dass ein Metallkomplexkatalysator auf einfache Weise aus einem Telomerisierungsgemisch abgetrennt werden kann, wenn die Abtrennung des Metallkomplexkatalysators an einer Membrane unter reaktionsnahen Bedingungen erfolgt, wobei insbesondere und eine gegen Alkalimetallverbindungen und gegen Lösemittel stabile. Membran eingesetzt wird. Auf diese Weise wird außerdem erreicht, dass die Aktivität des Metallkomplexkatalysators weitestgehend aufrechterhalten werden kann und der Metallkomplexkatalysator weitestgehend abgetrennt werden kann.

Gegenstand der Erfindung ist demnach ein Verfahren zur Abtrennung eines Metall-Komplexkatalysators aus einem Reaktionsgemisch erhalten aus einer Telomerisation, welches dadurch gekennzeichnet ist, dass der Metallkomplexkatalysator an zumindest einer Membran abgetrennt wird, die für Moleküle mit einer Molmasse bis 1000 g/mol durchlässig sind, wobei im Reaktionsgemisch vorhandener freier Ligand, ausgewählt aus Organophosphor- oder Carben-Ligand, an der Membran abgetrennt und in die Telomerisation zurückgeführt wird, und wobei die Membran ausgewählt ist aus Membranen, die als trennaktive Schicht eine alkali- und lösemittelstabile Nanofiltrationspolymerschicht aus Polyimiden (PI), aromatischen Polyamiden (PA), Polyamidimiden (PAI), Polybenzimidazolen, Acrylonitril/ Glycidylmethacrylat (PANGMA), Polybenzimidazolonen, Polyacrylnitril (PAN), Polyarylethersulfonen, Polyestern, Polyetheretherketonen (PEEK), Polycarbonaten (PC), Polytetrafluorethylen, Polybenzimidazol (PBI), Polyvinylidenfluorid (PVDF), Polypropylen (PP), Polydimethylsiloxan (PDMS), aufweisen, oder deren trennaktive Schicht aus Polymeren mit intrinsischer Milcroporosität (PIM) aufgebaut ist, oder deren trennaktive Schicht über eine hydrophobierte keramische Membran aufgebaut ist.

Das erfindungsgemäße Verfahren hat den Vorteil, dass die Aktivität des abgetrennten Katalysators weitestgehend erhalten bleibt. Dies liegt möglicherweise daran, dass eine thermische Belastung des Katalysatorsystems, wie sie bei thermischen Trennverfahren häufig zu beobachten ist, vermieden wird. Das Verfahren ist zudem einfach und vermeidet den Einsatz von Fremdstoffen, wie er bei Extraktions- oder Adsorptionsverfahren notwendig ist, welche die Gefahr der Verunreinigung des Produktes birgt, wenn der abgetrennte Katalysator der Telomerisation wieder zugeführt wird.

Nachfolgend wird das erfindungsgemäße Verfahren beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind nachfolgend Bereiche, allgemeine Formeln oder Verbindungsklassen angegeben, so sollen diese nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können.

Das erfindungsgemäße Verfahren zur Abtrennung eines Metall-Komplexkatalysators aus einem Reaktionsgemisch erhalten aus einer Telomerisation, zeichnet sich dadurch aus, dass der Metallkomplexkatalysator an zumindest einer Membran abgetrennt wird. Falls in dem erfindungsgemäß zu behandelnden Reaktionsgemisch der Telomerisation neben dem Metallkomplexkatalysator noch freie Liganden, insbesondere freie Carben- oder Organophosphor-Liganden vorhanden sind, kann es vorteilhaft sein, wenn auch diese freien Liganden an der zumindest einen Membran abgetrennt wird. Um eine Abtrennung des Metallkomplexkatalysators und gegebenenfalls vorhandener freier Liganden zur erreichen, wird vorzugsweise eine Membran eingesetzt, die für das Telomerisationsprodukt besser durchlässig ist als für den Metall-Komplexkatalysator und vorzugsweise auch besser als für eventuell vorhandene freie Liganden. Die Eignung von Membranen kann auf einfache Weise durch Vorversuche ermittelt werden, bei denen Testlösungen von Komplexkatalysatoren und/oder freien Liganden und Telomerisationsprodukt über die zu testende Membran gefahren werden und anschließend das erhaltene Permeat und Retentat analysiert werden. Besonders bevorzugt werden in dem erfindungsgemäßen Verfahren Membrane eingesetzt, die auf Grund ihrer chemischen oder physikalischen Eigenschaften geeignet sind, den Metallkomplexkatalysator und/oder freien Ligand (insbesondere Carben- und/oder Organophosphor-Ligand) im Vergleich zum Telomerisationsprodukt in einem Maße von zumindest 60 % zurückzuhalten.

Eine weitere Voraussetzung für die Verwendbarkeit der Membran besteht darin, dass die Membran stabil gegenüber allen in der Telomerisations-Reaktionsmischung vorhandenen Verbindungen, insbesondere gegenüber gegebenenfalls vorhandenen Lösemitteln und basischen Verbindungen, insbesondere Verbindungen der Alkalimetalle, sein sollte. Vorzugsweise werden deshalb Membrane eingesetzt, die als trennaktive Schicht eine alkali- und lösemittelstabile Nanofiltrationspolymerschicht aus einem Material, ausgewählt aus Polyimiden (PI), aromatischen Polyamiden (PA), Polyamidimiden (PAI), Polybenzimidazolen, Acrylonitril/Glycidylmethacrylat (PANGMA), Polybenzimidazolonen, Polyacrylnitril (PAN), Polyarylethersulfonen, Polyestern, Polyetheretherketonen (PEEK), Polycarbonaten (PC), Polytetrafluorethylen, Polyvinylidenfluorid (PVDF), Polypropylen (PP), Polydimethylsiloxan (PDMS) und anderen, wie sie z. B. in EP 0 781 166 oder DE 103 08 111 und in "Membranes" von I. Cabasso, Encyclopedia of Polymer Science and Technology, John Wiley and Sons, New York, 1987, beschrieben werden, aufweist oder aus einem dieser Materialien besteht. Besonders bevorzugt werden als Membrane solche eingesetzt, die PDMS oder Polyamidimid aufweisen oder aus diesen Materialien bestehen. Als Membrane können in dem erfindungsgemäßen Verfahren auch solche eingesetzt werden, deren trennaktive Schicht aus Polymeren mit intrinsischer Mikroporosität (PIM) aufgebaut ist, oder deren trennaktive Schicht über eine hydrophobierte keramische Membran aufgebaut ist. Solche mit Silanen hydrophobisierte keramische Membrane werden z. B. in DE 103 08 111 beschrieben.

Die Ausschlussgrenze (Molekulargewichts-Cutoff, MWCO) von bevorzugt eingesetzten Membrane liegt unter 1000 g/mol, vorzugsweise unter 600 g/mol, besonders bevorzugt unter 400 g/mol. Solche Membrane sind z. B. von HITK, Hermsdorf oder GMT, Rheinfelden zu beziehen. Die Ausschlussgrenze gibt an, bis zu welchen Molmassen in g/mol Moleküle die Membran passieren können.
Neben den oben genannten Materialien können die Membrane weitere Materialien aufweisen. Insbesondere können die Membrane Stütz- oder Trägermaterialien aufweisen, auf die die trennaktive Schicht aufgebracht ist. Bei solchen Verbundmembranen liegt neben der eigentlichen Membran noch ein Stützmaterial vor. Eine Auswahl von Stützmaterialien beschreibt EP 0 781 166, auf welches explizit verwiesen wird. Weiterhin können in der erfindungsgemäß einsetzbaren Membran Verstärkungsmaterialien vorhanden sein, wie z. B. Partikel von anorganischen Oxiden oder anorganische Fasern, wie z. B. Keramik- oder Glasfasern, die die Stabilität der Membrane, insbesondere gegenüber Druckschwankungen oder hohen Druckdifferenzen, erhöhen.

Besonders bevorzugt werden bei dem erfindungsgemäßen Verfahren solche Membranen eingesetzt, bei denen der Löslichkeitsparameter des Telomerisationsproduktes, bei Verwendung eines Alkohols als Telogen insbesondere des bei der Telomerisation erhaltenen Ethers sich in Bezug auf den Löslichkeitsparameter (J.M. Prausnitz, Molecular Thermodynamics of Fluid-Phase Equilibria, Prentice-Hall, NJ, 1969, p298) der eingesetzten Membran um zumindest ±50 √(kJ/m³), bevorzugt um zumindest ±50 √(kJ/m³) aber vorzugsweise um nicht mehr als ±500 √(kJ/m³), vorzugsweise um nicht mehr als ±400 √(kJ/m³) unterscheidet.

Das molare Volumenverhältnis der Liganden, insbesondere der Carben- oder Organophosphor-Liganden (frei oder im Komplex gebunden) zu dem Hauptprodukt der Telomerisation, bei Verwendung von Alkohol als Telogen also insbesondere dem Ether, sollte vorzugsweise größer-gleich 1,5, bevorzugt größer-gleich 3,0 und besonders bevorzugt größer gleich 3,5 betragen. Durch den großen molaren Volumenunterschied wird eine besonders gute Trennung von Ligand und Hydroformylierungsprodukt an der Membran erreicht. Die Löslichkeitsparameter und Molekularvolumen können wie in EP 0 781 166 B1, insbesondere in Abschnitt [0053] und den fortfolgenden Abschnitten sowie in der dort zitierten Literatur beschrieben, bestimmt werden.

Die Membranen werden in dem erfindungsgemäßen Verfahren vorzugsweise in Form von Membranmodulen eingesetzt. In diesen Modulen werden die Membranen so angeordnet, dass die Retentatseite der Membranen derart überströmt werden können, dass die Konzentrationspolarisation der abgetrennten Komponenten (die Anreicherung der abgetrennten Komponenten an der Membran), hier Katalysator-Ligandsystem, entgegengewirkt und außerdem die nötige treibende Kraft (Druck) aufgeprägt werden kann. Das Permeat wird im Permeatsammelraum auf der Permeatseite der Membranen zusammengeführt und aus dem Modul abgeführt. Gängige Membranmodule für Polymermembranen weisen die Membrane in Form von Membranscheiben, Membrankissen oder Membrantaschen auf. Gängige Membranmodule für Membranen auf Basis von Keramikträgem weisen diese in Form von Rohrmodulen auf. Im erfindungsgemäßen Verfahren werden die Membrane vorzugsweise in Form von Membranmodulen eingesetzt, die Membranmodule mit offenkanaligen Kissenmodulsystemen, bei denen die Membranen zu Membrantaschen oder -kissen thermische verschweißt oder verklebt sind, oder offenkanaligen (Wide-spacer) Wickelmodulen, bei denen die Membranen zu Membrantaschen oder Membrankissen verklebt oder verschweißt sind und mit Abstandshaltern um ein Permeatsammelrohr aufgewickelt sind, oder die Membranmodule in Rohrmodulen aufweisen. Membranmodule, die offenkanalige Zuflusssysteme aufweisen, bei denen die Membrane thermisch zu Membrantaschen oder Membrankissen verschweißt oder verklebt sind, sind z. B. bei der Firma Solsep, Apeldoorn (NL) und der Firma MET, London (UK) unter dem Namen SR-5 bzw. Starmem 240, das beispielsweise aus dem Polyimid mit dem Handelsnamen P84 der Degussa AG, Düsseldorf hergestellt werden kann, zu beziehen. Membranmodule, die Rohrmembranen auf einem keramischen Träger aufweisen, sind z. B. bei der Firma Inocermic, Schmalkalden zu beziehen.

Zur Vermeidung von Ablagerungen auf der Membran wird das Verfahren vorzugsweise so durchgeführt, dass die Membrantrennschritte, insbesondere der erste Membrantrennschritt mit einer Überströmgeschwindigkeit an der Membran von 0,1 bis 15 m/sec, bevorzugt 0,2 bis 4 m/sec, vorzugsweise von 0,3 bis 1 m/sec durchgeführt werden/wird.

Das erfindungsgemäße Verfahren kann unter Einsatz von einer, zwei oder mehreren Membran(en) oder unter Einsatz von einem, zwei oder mehreren Membranmodul(en) durchgeführt werden. Je nach Trennleistung der Membran und gewünschtem Rückhalt kann durch die Hintereinanderschaltung mehrerer Membrane bzw. Membranmodule der gewünschte Rückhalt erzielt werden. Die Hintereinanderschaltung kann in der Weise erfolgen, dass entweder das Retentat oder Permeat, vorzugsweise das Permeat einer ersten Membranabtrennung als Zulauf in eine weitere Membranabtrennung geleitet wird. Die auf die erste erfindungsgemäße Membranabtrennung gegebenenfalls folgende(n) weiteren Membranabtrennung(en) kann/können unter den gleichen Bedingungen wie die erste Membranabtrennung oder unter anderen Bedingungen, insbesondere anderen Temperaturen oder Drücken, durchgeführt werden.

Vorzugsweise wird das erfindungsgemäße Verfahren so betrieben, dass das Telomerisierungsgemisch als Zulaufstrom (Feed) auf die Membran gefahren und der Retentatstrom teilweise auf die Membran zurückgeführt wird. Dabei wird der Teilstrom, der auf die Membran zurückgeführt wird, mit dem Feed vereinigt. Der Teil des Retentatstroms, der nicht auf die Membran zurückgeführt wird, wird entweder als Zulaufstrom für eine oder mehrere nachfolgende Abtrennstufen verwendet oder aber in die Reaktion zurückgeführt.

Vorzugsweise beträgt das Volumenstrom-Verhältnis von Permeatstrom zu Zulaufstrom (ohne rückgeführtem Retentat) 1 zu 5 bis 1 zu 20, bevorzugt von 1 zu 7,5 bis 1 zu 12,5 und besonders bevorzugt von 1 zu 9 bis 1 zu 11. Das Volumenstromverhältnis kann über eine Veränderung des Differenzdrucks in Verbindung mit einer auf den erzeugten Permeatvolumenstrom bezogene Mengenregelung im Retentat eingestellt werden.

Es kann vorteilhaft sein, wenn der Volumenstrom, der über die Membran geführt wird, deutlich größer ist als der Volumenstrom des Permeatstroms, da auf diese einfache Weise eine hohe Überströmgeschwindigkeit an der Membran eingestellt werden kann. Vorzugsweise beträgt das Volumenstromverhältnis des auf die Membran geführten Stroms (Zulaufstrom inklusive rückgeführtem Retentat) zu Permeatstrom von 100 bis 10000 zu 1, bevorzugt von 500 bis 5000 zu 1 und besonders bevorzugt von 750 bis 1250 zu 1. Es wird also vorzugsweise ein relativ hoher Volumenstrom im Kreis über die Membran geführt. Die Größe des Teils des Retentatstroms, der in die Reaktion zurückgeführt oder einer weiteren Abtrennung zugeführt wird, ergibt sich aus der Differenz von Feedstrom (ohne rückgeführtem Retentat) und Permeatstrom.

Das erfindungsgemäße Abtrennverfahren kann als Druck getriebener Prozess erfolgen. Die Membranabtrennung wird vorzugsweise so durchgeführt wird, dass ein Druckunterschied von Retentat- zu Permeatseite von mindestens 0,5 MPa, vorzugsweise von 0,5 bis 10 MPa, bevorzugt von 1 bis 5 MPa vorliegt. Wird der Mindestdruckunterschied deutlich unterschritten, wird der transmembrane Fluss zu gering. Übersteigt der Differenzdruck den Wert von 10 MPa deutlich, so beginnen die meisten Membranen zu kompaktieren was ebenfalls zu einer Verringerung des transmembranen Flusses führt.

Das an der Membran erhaltene Permeat weist vorzugsweise eine Zusammensetzung auf, bei der der Anteil an Metallkomplexkatalysator und/oder an freiem Organophosphor-Ligand zumindest 50 %, bevorzugt zumindest 70 %, besonders bevorzugt zumindest 80 % und ganz besonders bevorzugt mehr als 90 % kleiner als im Retentat ist .

Das Permeat, welches gemäß dem Verfahren der vorliegenden Erfindung erhalten wird, kann auf herkömmliche Weise aufgearbeitet werden. So kann das Permeat einer thermischen Trennstufe, z. B. realisiert durch eine oder mehrere thermische Trennapparaturen, wie z. B. Dünnschichtverdampfer, Fallfilmverdampfer, Flashverdampfer oder Destillationskolonnen, zugeführt werden. Als Kopfprodukt wird das Telömerisationsprodukt sowie eventuell nicht umgesetzte Kohlenwasserstoffe, wie z. B. Diene, Olefine oder Aliphaten, gegebenenfalls nicht umgesetzte Telogen und gegebenenfalls bei der Telomerisierung eingesetztes Lösemittel, welches eine Siedetemperatur im Bereich der Telomerisierungsprodukte oder niedriger hat, erhalten, welches einer weiteren Aufarbeitung zugeführt werden kann. Als Sumpfprodukt dieser ersten Trennapparatur wird ein Gemisch erhalten, welches den Komplexkatalysator und/oder freien Liganden, gegebenenfalls ein höher als das Telomerisationsprodukt siedendes Lösemittel sowie während der Telomerisation entstandene Hochsieder enthält. Dieses Sumpfprodukt wird, vorzugsweise nach einer Ausschleusung eines Teils der Hochsieder, die thermische oder mittels einer (Membran-)Filtration erfolgen kann, in die Telomerisation zurückgeführt.
Das Permeat, welches gemäß dem Verfahren der vorliegenden Erfindung erhalten wird, kann aber auch einer wie im Stand der Technik beschriebenen Extraktion, Fällung oder Adsorption zugeführt werden.

Um die Verluste an Katalysator, insbesondere an aktivem Metallkomplexkatalysator möglichst gering zu halten, wird das Verfahren vorzugsweise so durch geführt, dass durch die Membranabtrennung an einer oder mehreren Membranen zumindest 80 %, vorzugsweise zumindest 90 % und besonders bevorzugt zumindest 95 % des ursprünglich in dem Telomerisationsgemisch vorhandenen Komplexmetallkatalysators aus dem Telomerisationsgemisch abgetrennt wird und in nachfolgenden Abtrennstufen die restlichen Anteile des Komplexkatalysators abgetrennt werden.

Es kann vorteilhaft sein, wenn aus dem Permeat, bevor dieses in die thermische Trennstufe geführt wird, ein Teil der Inhaltsstoffe entfernt wird. Insbesondere kann es vorteilhaft sein, solche Inhaltsstoffe aus dem Permeat abzutrennen, die bei den Druckbedingungen, bei denen die thermische Trennstufe betrieben wird, gasförmig vorliegen. Solche Inhaltsstoffe können z. B. nicht umgesetzte Kohlenwasserstoffe oder nicht umgesetzte Telogene sein. Zur Abtrennung dieser Inhaltsstoffe wird das Permeat bevorzugt einer Entgasung zugeführt, in welcher das Permeat auf einen geringeren Druck, vorzugsweise einen Druck, der gleich oder maximal 10 % höher als der Druck in der thermischen Trennstufe ist, entspannt wird. Die nach der Entspannung gasförmig vorliegenden Stoffe werden abgetrennt und können aufgearbeitet oder entsorgt werden oder aber direkt in die Reaktion zurückgeführt werden. Die verbleibenden, weiterhin flüssig vorliegenden Bestandteile des Permeats werden dann der thermischen Trennstufe zugeführt.

Durch die gekoppelte Abtrennung von Komplexkatalysator und/oder freiem Liganden in der Membranabtrennung und anschließender konventioneller Abtrennung wird erreicht, dass der Komplexkatalysator im Wesentlichen vollständig aus dem Telomerisationsgemisch auf relativ schonende Weise abgetrennt werden kann und deshalb zum überwiegenden Teil in der aktiven Form dem Prozess wieder zugeführt werden kann. Der bei der thermischen Abtrennung eventuell entstehende inaktive Katalysator kann gemeinsam mit den Hochsiedern ausgeschleust werden und z. B. durch Aufarbeitung zum reinen elementaren Metall zurückgewonnen werden. Der in der Membranabtrennung und gegebenenfalls in der nachfolgenden konventionellen Abtrennung abgetrennte Metallkomplexkatalysator kann in die Telomerisation zurückgeführt werden. Ebenso kann gegebenenfalls im Reaktionsgemisch vorhandener freier Ligand, vorzugsweise ausgewählt aus Organophosphor- oder Carben-Ligand an der Membran und gegebenenfalls in einer nachfolgenden konventionellen Abtrennung abgetrennt und in die Telomerisation zurückgeführt werden.

In einer besonders bevorzugten Ausführungsform des erfindungsgemäßen Verfahrens wird das Telomerisationsgemisch unter Bedingungen, die sich in Bezug auf Druck und Temperatur (in °C) maximal von 0 bis 50 %, vorzugsweise von 0 bis 30 % und bevorzugt von 0 bis 10 % von den Reaktionsbedingungen der Telomerisation unterscheiden, auf die Membran gefahren. Besonders bevorzugt wird das Telomerisationsgemisch unter Bedingungen, die in Bezug auf Druck und Temperatur maximal kleiner 0 bis 50 %, vorzugsweise kleiner 0 bis 30 %, bevorzugt kleiner 0 bis 10 % und besonders bevorzugt gar nicht von den Reaktionsbedingungen der Telomerisation unterscheiden, auf die Membran gefahren.

Als Telomerisationsgemische können in dem erfindungsgemäßen Verfahren alle bekannten Telomerisationsgemische, wie sie z. B. im oben genannten Stand der Technik erhalten werden, eingesetzt werden.

Als Reaktionsgemisch aus einer Telomerisation wird vorzugsweise ein Telomerisationsgemisch eingesetzt, welches durch Telomerisation von nicht cyclischen Olefinen mit mindestens zwei konjugierten Doppelbindungen mit mindestens einem Nucleophil (Telogen) unter Verwendung eines Katalysators enthaltend ein Metall der Gruppe 8, 9 oder 10 des Periodensystems der Elemente, erhalten wird.

In einer bevorzugten Telomerisation können als Einsatzstoffe die reinen nicht cyclischen Olefine mit konjugierten Doppelbindungen, Gemische verschiedener solcher Olefine oder Gemische eines oder mehrerer der genannten Olefine mit anderen Kohlenwasserstoffen eingesetzt werden. Bevorzugt wird als Einsatzstoff ein Gemisch von Kohlenwasserstoffen eingesetzt, das die nicht cyclischen Olefine, vorzugsweise ein nicht cyclisches Olefin mit mindestens zwei konjugierten Doppelbindungen, in Mischung mit anderen Kohlenwasserstoffen aufweist.

Besonders bevorzugt werden in der Telomerisation als nicht cyclische Olefine mit konjugierten Doppelbindungen 1,3-Butadien und/oder Isopren jeweils entweder als Reinstoff, Gemisch der Reinstoffe oder im Gemisch eines oder beider Olefine mit anderen Kohlenwasserstoffen eingesetzt. Ganz besonders wird in der Telomerisation ein Gemisch als Einsatzstoff eingesetzt, welches zu über 90 Gew.-% C₄-Kohlenwasserstoffe enthält und als nicht cyclisches Olefin 1,3-Butadien aufweist.

Als Einsatzstoffe für die Telomerisation sind besonders bevorzugt 1,3-Butadien-reiche Kohlenwasserstoffströme geeignet. Als Kohlenwasserstoffstrom kann insbesondere ein C₄-Kohlenwasserstoffschnitt eingesetzt werden. Die Kohlenwasserstoffströme können vorzugsweise z. B. Mischungen von 1,3-Butadien mit anderen C₄- und C₃- oder C₅-Kohlenwasserstoffen sein. Solche Mischungen fallen beispielsweise bei Spalt(Crack)-Prozessen zur Produktion von Ethylen und Propylen, in denen Raffineriegase, Naphtha, Gasöl, LPG (liquified petroleum gas), NGL (natural gas liquid) usw. umgesetzt werden, an. Die bei den Prozessen als Nebenprodukt anfallenden C₄-Schnitte können neben 1,3-Butadien, Monoolefine (1-Buten, cis-But-2-en, trans-But-2-en, Isobuten), gesättigte Kohlenwasserstoffe (n-Butan, Isobutan), acetylenisch ungesättigte Verbindungen (Ethylacetylen (Butin), Vinylacetylen (Butenin), Methylacetylen (Propin)) sowie allenisch ungesättigte Verbindungen (hauptsächlich 1,2-Butadien) enthalten. Weiterhin können diese Schnitte geringe Mengen an C₃- und C₅-Kohlenwasserstoffen enthalten. Die Zusammensetzung der C₄-Schnitte ist abhängig vom jeweiligen Spaltverfahren, den Betriebsparametern und dem Einsatzstoff. Die Konzentrationen der einzelnen Komponenten liegen typischerweise in folgenden Bereichen:

| Komponente | Massen-% |
|---|---|
| 1,3- Butadien | 25 - 70 |
| 1-Buten | 9 - 25 |
| 2-Butene | 4 - 20 |
| Isobuten | 10 - 35 |
| n-Butan | 0,5 - 8 |
| Isobutan | 0,5 - 6 |
| ∑ acetylenischer Verbindungen | 0,05 - 4 |
| 1,2-Butadien | 0,05 - 2 |

In der bevorzugten Telomerisation, deren Reaktionsgemisch in dem erfindungsgemäßen Verfahren eingesetzt wird, werden vorzugsweise Kohlenwasserstoffgemische mit einem 1,3-Butadiengehalt von größer 35 Massen-% eingesetzt.

Die Einsatzkohlenwasserstoffe können häufig Spuren von Sauerstoff-, Stickstoff-, Schwefel-, Halogen-, insbesondere Chlor- und Schwermetallverbindungen aufweisen, die im erfindungsgemäßen Verfahren störend sein könnten. Es ist daher zweckmäßig, diese Stoffe zunächst abzutrennen. Störende Verbindungen können z. B. Kohlendioxid oder Carbonylverbindungen, wie z. B. Aceton oder Acetaldehyd sein. Die Abtrennung dieser Verunreinigungen kann z. B. durch Wäschen, insbesondere mit Wasser oder wässrigen Lösungen, oder durch Adsorption erfolgen. Durch eine Wasserwäsche können hydrophile Komponenten, wie beispielsweise Stickstoffkomponenten, aus dem Kohlenwasserstoffgemisch ganz oder teilweise entfernt werden. Beispiele für Stickstoffkomponenten sind Acetonitril oder N-Methylpyrrolidon (NMP). Auch Sauerstoffverbindungen können zum Teil über eine Wasserwäsche entfernt werden. Die Wasserwäsche kann direkt mit Wasser durchgeführt werden oder aber mit wässrigen Lösungen, die z. B. Salze wie z. B. NaHSO₃ aufweisen können (US 3,682,779, US 3,308,201, US 4,125,568, US 3,336,414 oder US 5,122,236).

Es kann vorteilhaft sein, wenn das Kohlenwasserstoffgemisch nach der Wasserwäsche einen Trocknungsschritt durchläuft. Die Trocknung kann nach den im Stand der Technik bekannten Verfahren durchgeführt werden. Beim Vorliegen von gelöstem Wasser kann die Trocknung z. B. unter Verwendung von Molekularsieb als Trocknungsmittel oder durch azeotrope Destillation durchgeführt werden. Freies Wasser kann durch Phasentrennung, z. B. mit einem Koaleszer, abgetrennt werden.

Adsorber können eingesetzt werden, um Verunreinigungen im Spurenbereich zu entfernen. Dies kann insbesondere dann vorteilhaft sein, wenn im Telomerisationsschritt Edelmetallkatalysatoren zum Einsatz kommen, die bereits auf Spuren von Verunreinigungen mit deutlicher Aktivitätsabnahme reagieren. Oftmals werden Stickstoff- oder Schwefelverbindungen über vorgeschaltete Adsorber entfernt. Beispiele für Adsorptionsmittel sind Aluminiumoxide, Molekularsiebe, Zeolithe, Aktivkohle oder mit Metallen imprägnierte Tonerden (z. B. US 4,571,445 oder WO 02/53685). Adsorptionsmittel werden von diversen Firmen vertrieben, beispielsweise von der Firma Alcoa unter dem Namen Selexsorb^{®}, von UOP oder von Axens, z. B. mit den Produktserien SAS, MS, AA, TG, TGS oder CMG.

Die Abtrennung von evtl. störenden acetylenisch ungesättigten Verbindungen aus dem in der Telomerisation eingesetzten Kohlenwasserstoffgemisch kann z. B. durch Extraktion erfolgen. Eine solche Extraktion ist lange bekannt und ist als Aufarbeitungsschritt ein integraler Bestandteil der meisten Anlagen, die 1,3-Butadien aus Crack-C₄ gewinnen. Ein Verfahren zur extraktiven Abtrennung von acetylenisch ungesättigten Verbindungen aus Crack-C₄, wird beispielsweise in Erdöl und Kohle-Erdgas-Petrochemie vereinigt mit Brennstoffchemie Bd. 34, Heft 8, August 1981, Seiten 343 - 346 beschrieben. Bei diesem Verfahren werden in einer ersten Stufe durch Extraktivdestillation mit wasserhaltigem NMP die mehrfach ungesättigten Kohlenwasserstoffe sowie die acetylenisch ungesättigten Verbindungen von den Monoolefinen und gesättigten Kohlenwasserstoffen abgetrennt. Aus dem NMP Extrakt werden die ungesättigten Kohlenwasserstoffe destillativ abgetrennt und aus dem Kohlenwasserstoffdestillat durch eine zweite Extraktivdestillation mit wasserhaltigem NMP die acetylenisch ungesättigten Verbindungen mit 4 C-Atomen abgetrennt. Bei der Aufarbeitung von Crack-C₄ wird durch zwei weitere Destillationen reines 1,3-Butadien abgetrennt, wobei als Nebenprodukte Methylacetylen und 1,2-Butadien anfallen.
Optional kann die Abtrennung von acetylenischen Verbindungen aus einem 1,3-Butadien-haltigen Strom unter Verwendung von einer oder mehreren ionischen Flüssigkeit(en) z. B. als Extraktionsmittel, erfolgen.

Die durch Extraktion erhaltenen Kohlenwasserstoffströme, die vorzugsweise weniger als 5-Gew.-% an acetylenischen Verbindungen enthalten, können besonders bevorzugt direkt als Einsatzstoff in der Telomerisation eingesetzt werden.

Die teilweise Entfernung der acetylenisch ungesättigten Verbindungen aus dem einzusetzenden Kohlenwasserstoffstrom kann aber durch Selektivhydrierung der acetylenisch ungesättigten Verbindungen in Gegenwart von Dienen und Monoolefine, z. B. an Kupferhaltigen, Palladium-haltigen oder Mischkatalysatoren durchgeführt werden.

Als acetylenisch ungesättigte Verbindungen, enthalten die in der Telomerisation eingesetzten Einsatzstoffe, insbesondere bei der Verwendung von 1,3-Butadien aufweisenden C₄-Kohlenwasserstoffgemischen, häufig insbesondere Vinylacetylen und 1-Butin.

Das im erfindungsgemäßen Verfahren eingesetzte Telomerisationsgemisch stammt bevorzugt aus einer Telomerisation, bei der als Katalysatorsystem Metallkomplexkatalysatoren, insbesondere Metallcarbenkomplexe, der Metalle Palladium (Pd), Eisen (Fe), Ruthenium (Ru), Osmium (Os), Kobalt (Co), Rhodium (Rh), Iridium (Ir), Nickel (Ni) oder Platin (Pt) eingesetzt werden. Als Liganden können z. B. Phosphorliganden, wie z. B. Phosphine, Phosphinine, Phosphinite oder Phosphite, wie z. B. Triphenylphosphine, und/oder Carbenliganden eingesetzt werden, wobei es auch vorteilhaft sein kann unterschiedliche Liganden gleichzeitig zu verwenden.

Bevorzugt werden bei der Telomerisation Palladiumverbindungen, insbesondere Palladiumcarbenkomplexe als Katalysatoren im Telomerisationsschritt eingesetzt. Die Liganden in den als Katalysator eingesetzten Metallkomplexkatalysatoren sind besonders bevorzugt trivalente Phosporverbindungen oder Carbene.
Besonders bevorzugt werden Metallkomplexkatalysatoren als Katalysator in der Telomerisation verwendet, die mindestens ein durch Heteroatome stabilisiertes Carben als Ligand aufweisen. Beispiele für derartige Liganden werden unter anderem in den Dokumenten DE 101 28.144, DE 101 49 348, DE 101 48 722, DE 100 62 577, EP 1 308 157 und WO 01/66248 beschrieben. Diese Dokumente und insbesondere die dort beschriebenen Liganden zählen zum Offenbarungsgehalt der vorliegenden Anmeldung. Darüber hinaus kann der aktive Komplex noch weitere Liganden aufweisen. Die Carbenliganden können offene Liganden oder cyclische Liganden sein.

Vorzugsweise wird in der Telomerisation ein Palladiumcarbenkomplex als Telomerisationskatalysator eingesetzt, der einen Carbenliganden der allgemeinen Formel (VIII) 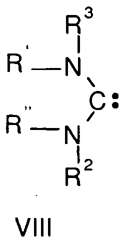
mit R², R", R' und R³ gleich oder unterschiedlich Wasserstoff oder Kohlenwasserstoffgruppen, wobei die Kohlenwasserstoffgruppen gleich oder unterschiedlich linear, verzweigt oder zyklische Reste ausgewählt aus der Gruppe von Alkylresten mit 1 bis 50 Kohlenstoffatomen, Alkenylresten mit 2 bis 50 Kohlenstoffatomen, Alkinylresten mit 2 bis 50 Kohlenstoffatomen und Arylresten mit 6 bis 30 Kohlenstoffatomen, in welchen zumindest ein Wasserstoffatom durch eine funktionelle Gruppe ersetzt sein kann,
und/oder R² und R" und/oder R' und R³ gleich Teil eines cyclischen Systems welches gleich oder unterschiedlich ist, welches ein Kohlenstoffgerüst mit von 2 bis 20 Kohlenstoffatomen und ein Stickstoffatom gemäß Formel VIII aufweist, wobei die Kohlenstoffatome von R² und R" und/oder R' und R³ nicht gezählt werden und wobei zumindest ein Wasserstoffatom in dem cyclischen System durch eine funktionelle Gruppe ersetzt sein kann und/oder zumindest ein Kohlenstoffatom des cyclischen Systems durch ein Heteroatom, ausgewählt aus der Gruppe bestehend aus S, P, O und N, ersetzt sein kann,
und/oder R² und/oder R" und/oder R' und/oder R³ sind durch eine Brücke aus 1 bis 20 Kohlenstoffatomen mit einem Liganden L verbunden, wobei die Kohlenstoffatome der Reste R², R", R' und R³ nicht mitgezählt werden,
und L ein weiterer Ligand ist, der für einen neutralen Zweielektronen-Donor, Teil eines cyclischen Systems und/oder einen anionischen Liganden steht, wobei die funktionellen Gruppen z. B. ausgewählt sein können aus den Gruppen: -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H und -PO₃H₂, wobei die Alkylgruppen z. B. 1 - 24 und die Arylgruppen z. B. 5 bis 24 Kohlenstoffatome beinhalten können, aufweist. Die Herstellung solcher Liganden kann z. B. DE 101 48 722 entnommen werden.

Bevorzugt wird in der Telomerisation, deren Rektionsgemisch im erfindungsgemäßen Verfahren auf die Membran gefahren wird, ein Palladiumcarbenkomplex als Telomerisationskatalysator eingesetzt, der einen Carbenliganden der allgemeinen Formeln (VIII) aufweist mit
- R²; R³:: gleich oder verschieden lineare, verzweigte, substituierte oder unsubstituierte cyclische oder alicyclische Alkylgruppen mit 1 bis 24 Kohlenstoffatomen, oder substituierte oder unsubstituierte, mono- oder polycyclische Arylgruppen mit 6 bis 24 Kohlenstoffatomen oder
mono- oder polycyclischer, substituierter oder unsubstituierter Heterocyclus mit 4 bis 24 Kohlenstoffatomen und mindestens einem Heteroatom aus der Gruppe N, O, S,
- R', R":: gleich oder verschiedenen
Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 - 24 und die Aryl- und Heteroarylgruppen 5 bis 24 Kohlenstoffatome beinhalten und die Reste R' und R" auch Teil eines verbrückenden aliphatischen oder aromatischen Rings sein können.

Ganz besonders bevorzugt werden Carbenliganden eingesetzt, die einen 5-Ring aufweisen. Vorzugsweise im erfindungsgemäßen Verfahren eingesetzte einen 5-Ring aufweisende Liganden sind z. B. solche der Formeln IX, X, XI und XII 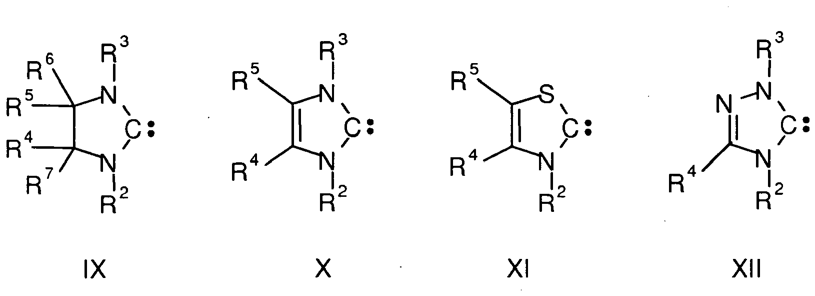 mit
- R²; R³:: gleich oder verschieden
lineare, verzweigte, substituierte oder unsubstituierte cyclische oder alicyclische Alkylgruppen mit 1 bis 24 Kohlenstoffatomen, oder
substituierte oder unsubstituierte, mono- oder polycyclische Arylgruppen mit 6 bis 24 Kohlenstoffatomen oder
mono- oder polycyclischer, substituierter oder unsubstituierter Heterocyclus mit 4 bis 24 Kohlenstoffatomen und mindestens einem Heteroatom aus der Gruppe N, O, S,
- R⁴, R⁵, R⁶, R⁷:: gleich oder verschiedenen
Wasserstoff, Alkyl, Aryl, Heteroaryl, -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂, wobei die Alkylgruppen 1 - 24 und die Aryl- und Heteroarylgruppen 5 bis 24 Kohlenstoffatome beinhalten und die Reste R' und R" auch Teil eines verbrückenden aliphatischen oder aromatischen Rings sein können.

Beispiele für Carbenliganden, die den allgemeinen Formeln IX oder X entsprechen, und Komplexe, die derartige Liganden enthalten, sind in der Fachliteratur bereits beschrieben (W. A. Herrmann, C. Köcher, Angew. Chem. 1997, 109, 2257; Angew. Chem. Int. Ed. Engl. 1997, 36, 2162; V.P.W. Böhm, C.W.K. Gstöttmayr, T. Weskamp, W.A. Herrmann, J. Organomet. Chem. 2000, 595, 186; DE 44 47 066).

Die Reste R² und R³ können insbesondere für einen mono- oder polycyclischen Ring stehen, der mindestens ein Heteroatom ausgewählt aus den Elementen Stickstoff, Sauerstoff und Schwefel enthält und gegebenenfalls weitere Substituenten ausgewählt aus den Gruppen -CN, -COOH, -COO-Alkyl-, -COO-Aryl-, -OCO-Alkyl-, -OCO-Aryl-, -OCOO-Alkyl-, -OCOO-Aryl-, -CHO, -CO-Alkyl-, -CO-Aryl-, -Aryl-, -Alkyl-, -O-Alkyl-, -O-Aryl-, -NH₂, -NH(Alkyl)-, -N(Alkyl)₂-, -NH(Aryl)-, -N(Alkyl)₂-, -F, -Cl, -Br, -I, -OH, -CF₃, -NO₂, -Ferrocenyl, -SO₃H, -PO₃H₂ aufweist. Die Alkylgruppen weisen 1 bis 24 und die Arylgruppen 5 bis 24 Kohlenstoffatome auf. In dem Fall, das Pd als Metall der 8. bis 10. Gruppe des Periodensystems verwendet wird, weist einer oder beide Liganden R² und R³, bevorzugt diese Bedeutungen auf.

Die Reste R², R³, R⁴, R⁵, R⁶ und/oder R⁷ können jeweils gleich oder verschieden sein und mindestens einen Substituenten aus der Gruppe -H, -CN, -COOH, -COO-Alkyl, -COO-Aryl, -OCO-Alkyl, -OCO-Aryl, -OCOO-Alkyl, -OCOO-Aryl, -CHO, -CO-Alkyl, -CO-Aryl, -Aryl, -Alkyl, -Alkenyl, -Allyl, -O-Alkyl, -O-Aryl, -NH₂, -NH(Alkyl), -N(Alkyl)₂, -NH(Aryl), -N(Alkyl)₂, -F, -Cl, -Br, -I, -OH, -CF₃, -NO₂, -Ferrocenyl, -SO₃H, -PO₃H₂ aufweisen, wobei die Alkylgruppen 1 bis 24, bevorzugt 1 bis 20, die Alkenylgruppen 2 bis 24, die Allylgruppen 3 bis 24 und die mono- oder polycyclischen Arylgruppen 5 bis 24 Kohlenstoffatome beinhalten. Die Reste R₄ bis R₆ können z. B. über (CH₂)- oder (CH)-Gruppen miteinander kovalent verknüpft sein.

Die Substituenten mit aciden Wasserstoffatomen können an Stelle der Protonen auch Metall-oder Ammoniumionen aufweisen.
Die Reste R² und R³ können besonders bevorzugt Reste sein, die sich von fünf- und sechsgliedrigen Heteroalkanen, Heteroalkenen und Heteroaromaten wie 1,4-Dioxan, Morpholin, γ-Pyran, Pyridin, Pyrimidin, Pyrazin, Pyrrol, Furan, Thiophen, Pyrazol, Imidazol, Thiazol und Oxazol ableiten. In der nachfolgenden Tabelle sind konkrete Beispiele für derartige Reste R² und R³ widergegeben. Darin bezeichnet ~ jeweils den Anknüpfungspunkt zum Fünfring-Heterozyklus.

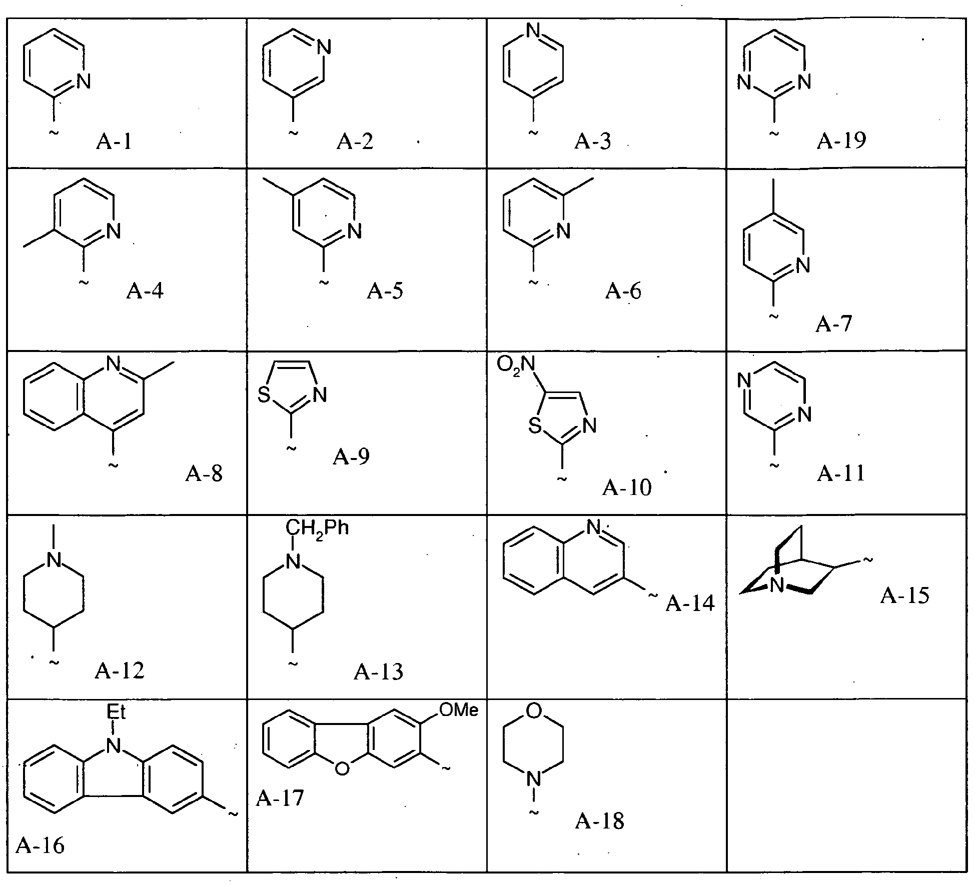

Im Rahmen dieser Erfindung werden unter Carben-Liganden sowohl freie Carbene, die als Ligand fungieren können, als auch an Metall koordinierte Carbene verstanden.
Das Katalysatormetall, insbesondere das als Katalysatormetall verwendete Palladium, aus dem die sich unter Reaktionsbedingungen die aktiven Katalysatoren bilden, kann auf verschiedene Weisen in den Prozess der Telomerisation eingebracht werden.

Das Metall (Palladium) kann
a) als Metall-Carbenkomplex (Palladium-Carbenkomplex), wobei das Metall (Palladium) bevorzugt in den Oxidationsstufen (II) oder (0) vorliegt oder
b) in Form von Metallverbindungen (Palladiumverbindungen als Vorstufen), aus denen in situ die Katalysatoren gebildet werden,
in den Prozess der Telomerisation eingebracht werden.

### Zu a)

Beispiele sind Palladium(0)carben-olefin-Komplexe, Palladium(0)dicarbenkomplexe und Palladium(II)dicarbenkomplexe, Palladium(0)carben-1,6-dien-Komplexe. Als 1,6-Dien können beispielsweise Diallylamin, 1,1'-Divinyltetramethyldisiloxan, 2,7-Octadienylether oder 2,7-Octadienylamine fungieren. Beispiele zeigen die nachfolgenden Fomeln I-a bis I-e.

| | | |
|---|---|---|
| 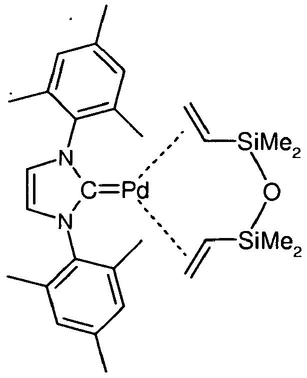 | 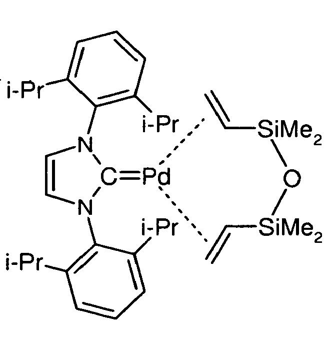 | 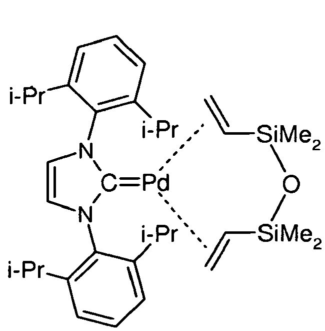 |
| I-a | I-b | I-c |
| 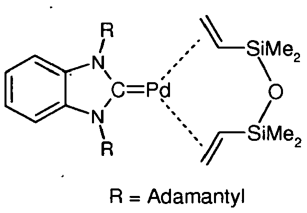 | 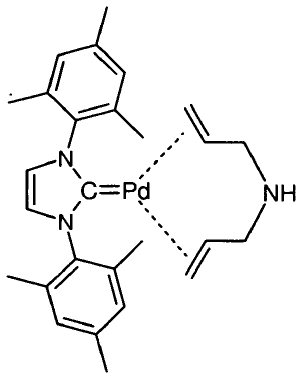 | 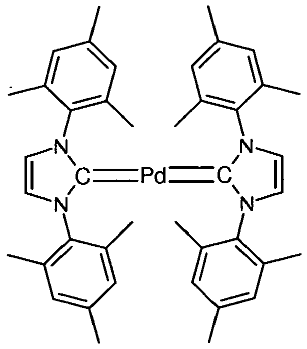 |
| I-d | I-e | I-f |
| 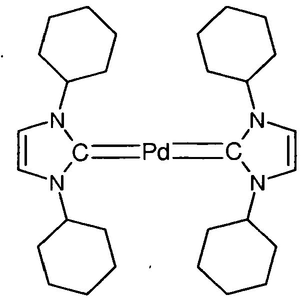 | 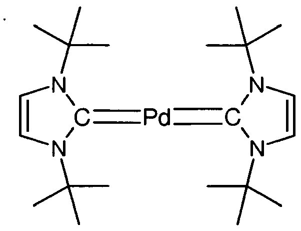 | 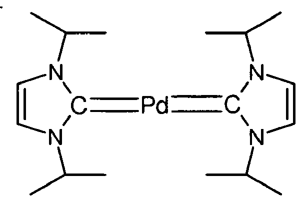 |
| 1-g | I-h | I-i |
| 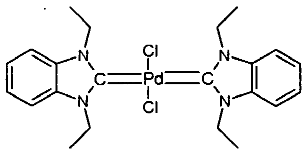 | 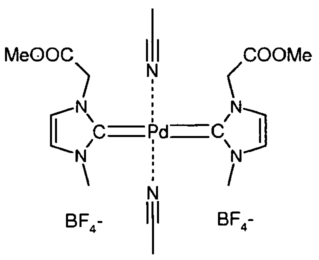 | 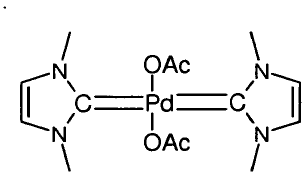 |
| I-j | I-k | I-l |

Die Carbenkomplexe des Palladiums können auf verschiedenste Weisen dargestellt werden. Ein einfacher Weg ist beispielsweise die Anlagerung von Carbenliganden oder der Austausch von Liganden an Palladiumkomplexen durch Carbenliganden. So sind beispielsweise die Komplexe I-f bis I-i durch Austausch der Phosphorliganden des Komplexes Bis(tri-o-tolylphosphin)palladium(0) erhältlich (T. Weskamp, W.A. Herrmann, J. Organomet. Chem. 2000, 595, 186).

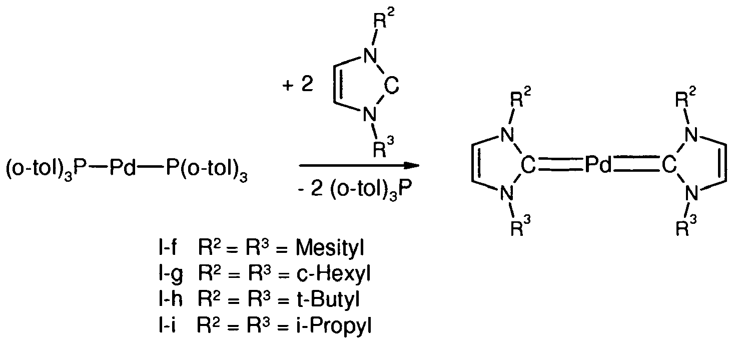

### Zu b)

Als Palladiumvorstufen können beispielsweise eingesetzt werden: Palladium(II)acetat, Palladium(II)chlorid, Palladium(II)bromid, Lithiumtetrachloropalladat, Palladium(II)acetylacetonat, Palladium(0)-dibenzylidenaceton-Komplexe, Palladium(II)propionat, Palladium(II)chloridbisacetonitril, Palladium(II)-bistriphenylphosphandichlorid, Palladium(II)chloridbisbenzonitril, Bis(tri-o-tolylphosphin)palladium(0) und weitere Palladium(0)-und Palladium(II)-Komplexe.

Die Carbene nach den allgemeinen Formeln IX und X können in Form freier Carbene oder als Metallkomplexe eingesetzt werden oder in situ aus Carbenvorstufen erzeugt werden.

Als Carbenvorstufen eignen sich beispielsweise Salze der Carbene gemäß den allgemeinen Formeln XIII und XIV, 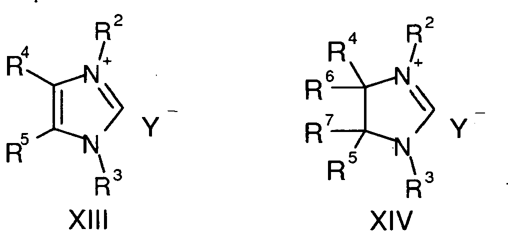 wobei R², R³, R⁴, R⁵, R⁶, R⁷ dieselbe Bedeutung haben wie in Formeln IX und X für eine einfach geladene anionische Gruppe oder entsprechend der Stöchiometrie anteilig für eine mehrfach geladene anionische Gruppe steht.

Beispiele für Y sind Halogenide, Hydrogensulfat, Sulfat, Alkylsulfonate, Arylsulfonate, Borate, Hydrogencarbonat, Carbonat, Alkylcarboxylate, Arylcarboxylate und Phenolate.

Aus den Salzen der Carbene können die entsprechenden Carbene beispielsweise durch Umsetzung mit einer Base freigesetzt werden.

Die Konzentration des Metallkomplexkatalysators, formal angegeben in ppm (Masse) an Palladium-Metall bezogen auf die Gesamtmasse, beträgt in dem Telomerisationsgemisch vorzugsweise von 0,01 ppm bis 1000 ppm, bevorzugt von 0,5 bis 100 ppm, besonders bevorzugt von 1 bis 50 ppm. Das Verhältnis [Mol/Mol] von Carben- oder Organophosphor-Ligand, vorzugsweise von Carben-Ligand zu Metall, insbesondere zu Pd beträgt bevorzugt von 0,01 : 1 bis 250: 1, besonders bevorzugt von 1 : 1 bis 100: 1 und ganz besonders bevorzugt von 1 : 1 bis 50 : 1. Neben den Carbenliganden können noch weitere Liganden, beispielsweise die oben genannten Organophosphor-Liganden, wie z. B. Triphenylphosphin, in dem Telomerisationsgemisch vorliegen.

Als Nukleophil (VII) werden in der Telomerisation bevorzugt Verbindungen der allgemeinen Formeln

R^{1a}-O-H (VIIa) oder (R^{1a})(R^{1b})N-H (VIIb)

oder

R^{1a}-COOH (VIIc)

worin R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe - CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ enthalten können, und wobei die Reste R^{1a} und R^{1b} über kovalente Bindungen miteinander verknüpft sein können, eingesetzt. Vorzugsweise werden als Nucleophile solche Verbindungen eingesetzt, bei denen die Reste R^{1a} und gegebenenfalls R^{1b} gleich Wasserstoff, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o- oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl sind.

Besonders bevorzugt werden in der Telomerisation, deren Reaktionsgemisch im erfindungsgemäßen Verfahren auf die Membran geführt wird, als Nucleophile (VII) Wasser, Alkohole, Phenole, Polyole, Carbonsäuren, Ammoniak und/oder primäre oder sekundäre Amine eingesetzt. Speziell sind dies:
- Wasser, Ammoniak,
- Monoalkohole und Phenole wie zum Beispiel Methanol, Ethanol, n-Propanol, Isopropanol, Allylalkohol, n-Butanol, i-Butanol, Octanol, 2-Ethylhexanol, Isononanol, Benzylalkohol, Cyclohexanol, Cyclopentanol oder 2,7-Octadien-1-ol, Phenol,
- Dialkohole wie zum Beispiel Ethylenglykol, 1,2-Propandiol, 1,3-Propandiol, 1,4-Butandiol, 1,2-Butandiol, 2,3-Butandiol und 1,3-Butandiol,
- Hydroxyverbindungen wie zum Beispiel α-Hydroxyessigsäureester,
- primäre Amine wie zum Beispiel Methylamin, Ethylamin, Propylamin, Butylamin, Octylamin, 2,7-Octadienylamin, Dodecylamin, Ethylendiamin oder Hexamethylendiamin,
- sekundäre Amine wie Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin oder
- Carbonsäuren wie Ameisensäure, Essigsäure, Propansäure, Butensäure, Isobutensäure, Benzoesäure, 1,2 Benzoldicarbonsäure (Phthalsäure).

Ganz besonders bevorzugt werden als Nucleophile (VII) in der Telomerisation Methanol, Ethanol, 2-Ethlyhexanol, Octanol, Octenol, Octadienol, Isopropanol, n-Propanol, Isobutanol, n-Butanol, Isononanol, Ameisensäure, Essigsäure, Propionsäure, n-Butansäure, iso-Butansäure, Benzoesäure, Phthalsäure, Phenol, Dimethylamin, Methylamin, Ammoniak und/oder Wasser eingesetzt. Vorteilhafterweise wird als Nucleophil Methanol eingesetzt.

Für das Verhältnis von Nucleophil zum in der Telomerisation umzusetzenden Ausgangsolefin mit mindestens zwei konjugierten Doppelbindungen ist die Anzahl der aktiven Wasserstoffatome im Telogen zu berücksichtigen. So hat beispielsweise Methanol ein aktives Wasserstoffatom, Ethylenglykol hat zwei, Methylamin hat zwei usw.

Pro Mol aktivem Wasserstoffatom des Nukleophils, das mit dem Ausgangsolefin reagieren kann, werden bevorzugt 0,001 Mol bis 10 Mol Ausgangsolefin in der Telomerisationsreaktion eingesetzt. Bei einer Reaktionsführung der Telomerisation mit einer flüssigen Phase wird ein Verhältnis von 0,1 Mol bis 2 Mol Ausgangsolefin pro Mol aktivem Wasserstoff besonders bevorzugt.

Es kann vorteilhaft sein, wenn die Telomerisation in Anwesenheit eines Lösemittels durchgeführt wird. Als Lösemittel für die Telomerisationsreaktion kann das eingesetzte Nucleophil, wenn es bei Reaktionsbedingungen als Flüssigkeit vorliegt, und/oder inerte organische Lösemittel eingesetzt werden. Bevorzugt wird der Zusatz von Lösemitteln bei Einsatz von Nucleophilen, die unter Reaktionsbedingungen als Feststoffe vorliegen oder bei Produkten, die unter den Reaktionsbedingungen der Telomerisation als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer Alkane (C₃-C₂₀), Cyclohexan, Cyclooctan, Ethylcyclohexan, Alkene und Polyene, Vinylcyclohexen, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon; Carbonsäureester wie zum Beispiel Essigsäureethylester, Ether wie beispielsweise Dipropylether, Diethylether, Dimethylether, Methyloctylether, Methyltertiärbutylether, Ethyltertiärbutylether 3-Methoxyoctan, Dioxan, Tetrahydrofuran, Anisol, Alkyl- und Arylether von Ethylenglycol, Diethylenglycol, Triethylenglycol, Tetraethylenglycol, Polyethylenglycol, Propylenglycol, Dipropylenglycol, Tripropylenglycol und Polypropylenglycol und andere polare Lösemittel wie zum Beispiel Sulfolan, Dimethylsulfoxid, Ethylencarbonat, Propylencarbonat und Wasser. Auch ionische Flüssigkeiten, beispielsweise Imidazolium- oder Pyridiniumsalze, können als Lösemittel eingesetzt werden. Die Lösemittel können allein oder als Mischungen verschiedener Lösemittel eingesetzt werden.

Oftmals ist es vorteilhaft, die Telomerisationsreaktion in Gegenwart von Basen durchzuführen. Bevorzugt werden basische Komponenten mit einem pK_{b}-Wert kleiner 7, insbesondere Verbindungen ausgewählt aus der Gruppe der Amine, Alkoholate, Phenolate, Alkalimetallsalze oder Erdalkalimetallsalze eingesetzt.

Als basische Komponente sind beispielsweise geeignet Amine wie Trialkylamine, die alicyclisch oder/und offenkettig sein können, Amide, Alkali- oder/und Erdalkalisalze aliphatischer oder/und aromatischer Carbonsäuren, wie Acetate, Propionate, Benzoate bzw. entsprechende Carbonate, Hydrogencarbonate, Alkoholate von Alkali- und/oder Erdalkalielementen, Phosphate, Hydrogenphosphate oder/und Hydroxide bevorzugt von Lithium, Natrium, Kalium, Calcium, Magnesium, Cäsium, Ammonium- und Phosphoniumverbindungen. Bevorzugt sind als Zusatz Hydroxide der Alkali- und Erdalkalielemente und Metallsalze des Nucleophils (VII) nach den allgemeinen Formeln IV, V oder VI.

ROMe IV

R¹R²NMe V

RCOOMe VI

Mit Me = einwertiges Metall oder einwertiges Metalläquivalent, R und R¹ mit der Bedeutung wie oben für R^{1a} angegeben und R² mit der Bedeutung wie oben für R^{1b} angegeben.

Vorzugsweise werden von der basischen Komponente von 0,01 Mol-% bis 10 Mol-% (bezogen auf das Ausgangsolefin), bevorzugt von 0,1 Mol-% bis 5 Mol-% und ganz besonders bevorzugt von 0,2 Mol-% bis 1 Mol-% eingesetzt.
Die Telomerisation kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Die Telomerisation wird im Hinblick auf eine hohe Raum-Zeit-Ausbeute vorzugsweise nicht bis zum vollständigen Umsatz des Einsatzolefins durchgeführt. Dies kann insbesondere dann vorteilhaft sein, wenn Einsatzolefin 1,3-Butadien ist. In diesem Fall ist es, insbesondere bei Einsatz von Methanol als Nucleophil, bevorzugt, den Umsatz auf maximal 95 %, besonders bevorzugt auf 88 %, zu begrenzen.

Die Temperatur, bei der die Telomerisationsreaktion ausgeführt wird, liegt vorzugsweise im Bereich von 10 bis 180 °C, bevorzugt im Bereich von 30 bis 120 °C und besonders bevorzugt im Bereich von 40 bis 100 °C. Die Reaktion wird vorzugsweise bei Normaldruck oder bei erhöhtem Druck, insbesondere bei einem erhöhten Druck von 0,15 bis 30 MPa, bevorzugt von 0,2 bis 12 MPa, besonders bevorzugt von 0,5 bis 6,4 MPa und ganz besonders bevorzugt von 1 bis 5 MPa, durchgeführt.

Das erfindungsgemäße Verfahren wird deshalb in der bevorzugten Ausführungsform, bei der die Membranabtrennung unter Bedingungen erfolgt, die im Bereich der Reaktionsbedingungen der Telomerisation liegen, vorzugsweise so durchgeführt, dass die Membranabtrennung bei einer Temperatur vorzugsweise von 5 bis 180 °C, bevorzugt von 15 bis 120 °C und besonders bevorzugt von 20 bis 100 °C durchgeführt wird. Ganz besonders bevorzugt wird die Membranabtrennung bei einer Temperatur von 80 bis 100 °C durchgeführt. Vorzugsweise erfolgt die Membranabtrennung aus dem selben Grund bei Normaldruck oder bei einem erhöhten Druck, insbesondere bei einem erhöhten Druck von 0,075 bis 30 MPa, bevorzugt von 0,1 bis 12 MPa, besonders bevorzugt von 0,25 bis 6,4 MPa und ganz besonders bevorzugt von 0,5 bis 5 MPa.

Das erfindungsgemäße Verfahren kann insbesondere zur Herstellung einer Verbindung gemäß der Formel II 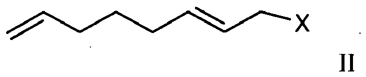 in der X ein Rest OR^{1a} oder NR^{1a}R^{1b} wobei R^{1a} und R^{1b} unabhängig voneinander ausgewählt sind aus Wasserstoff, einer linearen, verzweigten oder cyclischen C₁ bis C₂₂-Alkylgruppe, einer Alkenylgruppe, einer Alkinylgruppe, einer C₅ bis C₁₈ Arylgruppe oder einer -CO-Alkyl-(C₁-C₈)-Gruppe oder einer -CO-Aryl-(C₅-C₁₀)-Gruppe, wobei diese Gruppen Substituenten ausgewählt aus der Gruppe -CN, -COOH, -COO-Alkyl-(C₁-C₈), -CO-Alkyl-(C₁-C₈), -Aryl-(C₅-C₁₀), -COO-Aryl-(C₆-C₁₀), -CO-Aryl-(C₆-C₁₀), -O-Alkyl-(C₁-C₈), -O-CO-Alkyl-(C₁-C₈), -N-Alkyl₂-(C₁-C₈), -CHO, -SO₃H, -NH₂, -F, -Cl, -OH, -CF₃, -NO₂ enthalten können, und wobei die Reste R^{1a} und R^{1b} über kovalente Bindungen miteinander verknüpft sein können, aus einem 1,3-Butadien-haltigen Kohlenwasserstoffstrom eingesetzt werden.

Das erfindungsgemäße Verfahren kann insbesondere zur Aufarbeitung von Telomerisationsgemischen eingesetzt werden, welche bei der Herstellung von Verbindungen der Formeln IIIa oder IIIb, 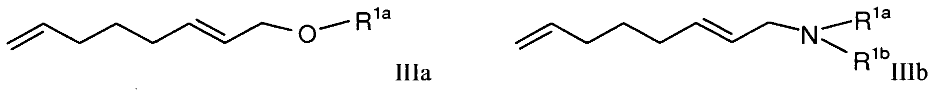 durch Umsetzung von 1,3-Butadien mit einem Nucleophil (VII) gemäß den Formeln VIIa, VIIb oder VIIc

R^{1a}-O-H VIIa

(R^{1a})(R^{1b})N-H VIIb

R^{1a}-COOH VIIc

erhalten werden, wobei R^{1a} und R^{1b} die oben genannte Bedeutung haben.

Besonders bevorzugt werden mit der Telomerisation Verbindungen der Formel II hergestellt, in denen das X für OR^{1a} oder NR^{1a}R^{1b} steht, mit
R^{1a} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o-oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl und/oder
R^{1b} gleich H, Methyl, Ethyl, n-Propyl, iso-Propyl, tert.-Butyl, n-Butyl, sec.-Butyl, Pentyl, Hexyl, Heptyl, Octyl, Octenyl, Octadienyl, Isononyl, 2-Ethylhexyl, n-Nonyl, Phenyl, m-, o-oder p-Methylphenyl, Naphtyl, 2,4-Di-tert.-butylphenyl, 2,6-Di-tert.-butylmethylphenyl, Hydrogencarbonyl, Methylcarbonyl, Ethylcarbonyl, Propylcarbonyl oder Phenylcarbonyl. Ganz besonders bevorzugt wird mit dem erfindungsgemäßen Verfahren eine Verbindung gemäß der Formel IIIa mit R^{1a} = Wasserstoff, Methyl, Ethyl, Phenyl oder Methylcarbonyl hergestellt. Die Verbindungen der Formeln IIIa und IIIb können sowohl in der cis- als auch in der trans-Form vorliegen.

Ganz besonders bevorzugt wird mit der Telomerisation ein 2,7-Octadienylderivat, insbesondere 1-Methoxyocta-2,7-dien, aus dem durch Hydrierung der beiden Doppelbindungen und anschließende Methanol-Abspaltung 1-Octen hergestellt werden kann, hergestellt.

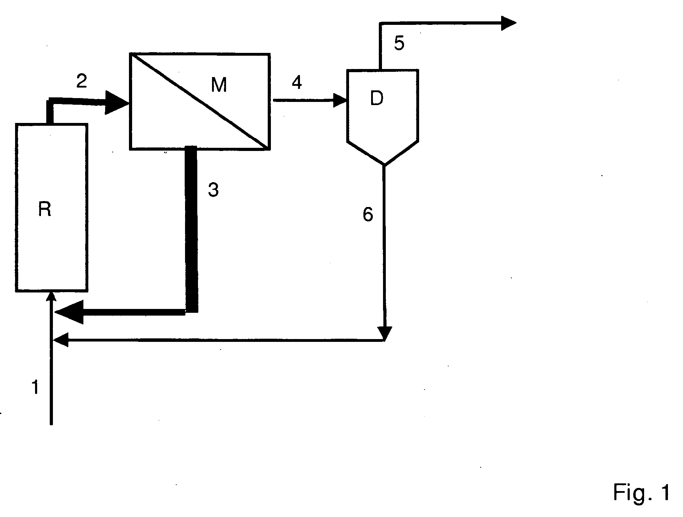

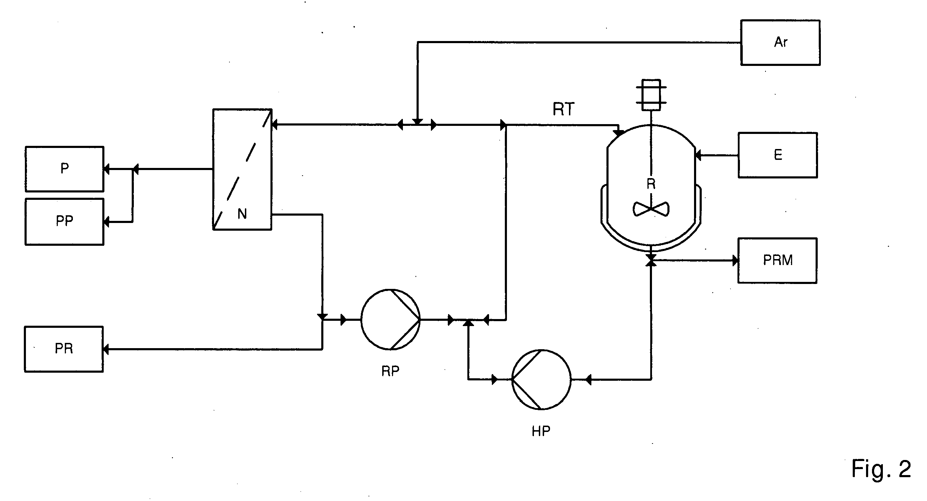

Die vorliegende Erfindung wird an Hand der Figuren Fig. 1 und 2 beispielhaft beschrieben, ohne dass die Erfindung auf diese Ausführungsform beschränkt sein soll.

In Fig. 1 ist eine schematische Abbildung einer Ausführungsform des erfindungsgemäßen Verfahrens dargestellt. Bei dieser Ausführungsform werden die Reaktanden 1 der Telomerisation und ein Rückführstrom 6 in den Reaktor R eingespeist, in dem die Telomerisation stattfindet. Der Reaktor kann dabei ein Rührkessel oder ein Rohrreaktor sein. Das Telomerisationsgemisch 2 wird direkt auf die Membran M geführt. Der an der Membran erhaltene Retentatstrom 3 wird in die Reaktion zurückgeführt. Der an der Membran M erhaltene Permeatstrom 4 wird in eine thermische Trennvorrichtung D, z. B. in einen Dünnschichtverdampfer, geführt. In diesem erfolgt eine Auftrennung in Telomerisationsprodukt und gegebenenfalls nicht umgesetzte Olefine, die als Strom 5 die thermische Trennvorrichtung verlassen, und einen Strom 6, der Hochsieder und in der Membranabtrennung nicht abgetrennten Komplexkatalysator und/oder freien Liganden enthält und in den Reaktor R zurückgeführt wird.
In Fig. 2 ist die Prozessführung in der Versuchsanlage gemäß Beispiel 1 schematisch dargestellt. Der Prozess besteht aus einer Reaktion in Reaktor R mit eingebautem Rührer und einer Nanofiltration N. Die gesamte Anlage kann mit Argon AR gespült und sauerstofffrei gestellt werden. Für die Reaktion wird das Eduktgemisch E, welches Palladiumverbindung und Ligand enthält, im Reaktor R vorgelegt und umgesetzt. Das Reaktionsprodukt wird in die Nanofiltration N überführt. Aus der Nanofiltration wird an der Membran das Permeat P erhalten, das vorwiegend aus Reaktionsprodukt besteht. Das bei der Nanofiltration anfallende Retentat RT, welches den Katalysator sowie den Liganden enthält, wird in den Reaktor zurückgeführt. Dadurch wird das abgetrennte Katalysator-Ligandgemisch aufkonzentriert.

Am Reaktorausgang kann eine Probe PRM zur Analyse der erhaltenen Reaktionsmischung genommen werden. Mittels einer Hochdruckpumpe HP wird das Reaktionsgemisch einem Kreislauf zugeführt, der in die Nanofiltration N führt. Die Rezirkulationspumpe RP sorgt für die nötige Überströmung der Membran. Permeatseitig wird aus der Nanofiltration das Permeat P abgezogen. In der Ablaufleitung für das Permeat P ist eine Probenentnahmemöglichkeit zur Entnahme einer Probe des Permeats PP vorhanden. In der Ablaufleitung des Retentats aus der Nanofiltration ist eine Probenentnahmevorrichtung vorhanden, mit welcher eine Probe des Retentats PR zu Analysezwecken entnommen werden kann.

Das folgende Beispiel soll die Erfindung näher erläutern, ohne den Schutzbereich einzuschränken, der sich aus den Patentansprüchen und der Beschreibung ergibt.

### Beisp iel 1

In einer wie in Fig. 2 dargestellten Versuchsanlage wurden für die Teloinerisierungsreaktion die Edukte (E) unter Sauerstoffausschluss durch vorherige Spülung mit Argon (Ar) mit folgender Zusammensetzung vorgelegt:
- 331,4 g Methanol
- 1170 g Crack C4 (davon 511,2 g 1,3 Butadien) (Oxeno Olefinchemie GmbH)
- 0,1040 g Palladiumacetylacetat (Umicore)
- 0,7658 g 1,3-Dimesitylimidazoliumchlorid (Degussa AG)
- 7,6 g Natrimmethylat (Aldrich)
- 13,42 g o-Kresol (Aldrich)
- 200,8 g Tripropylenglycol (Aldrich)

Nachdem alle Edukte bei 80 °C im Reaktor vorgelegt waren, wurde zuletzt der Pd-Katalysator mit Ligand (1,3-Dimesitylimidazoliumchlorid) mit einer Restmenge (100 g) an Methanol zugegeben und damit die Reaktion gestartet. Mittels einer stündlichen Probennahme am Reaktor wurde der Reaktionsverlauf mittels GC verfolgt.

Die Reaktion wurde durch Abkühlung auf 25 °C nach 240 Minuten abgebrochen. Anschließend wurde das Reaktionsproduktgemisch mit dem gelösten Katalysatorsystem über die Nanofiltrationsanlage (N) im Rücklauf geführt. Hierbei handelte es sich um eine Einheit, die über eine Hochdruckpumpe gespeist wird und die den benötigten transmembranen Druck im System aufbaut. Von dort gelangte das zu filtrierende Medium über eine Rezirkulationspumpe in das Membranmodul "Memcell" der Firma Osmota mit einer Fläche von 80 cm². In diesem Modul war eine Membran des Typs PDMS (6 % Strahlenvemetzt)-PPSU (Polyphenylensulfon), GKSS, Geesthacht eingebaut, die mit einem transmembranen Druck von 30 bar mit 1,7 m/s überströmt wurde. Zur Verhinderung des Gasaustrittes wurde auf der Permeatseite ein Druck von 1,7 bar eingestellt, wodurch bei einem transmembranen Druck von 30 bar auf der Retentatseite 31,7 bar eingestellt wurde.

Während des Rücklaufbetriebs über die Nanofiltration wurde dem System über die Membran Permeat, das vorwiegend aus Reaktionsprodukt bestand, entnommen. Der Katalysator sowie der Ligand 1,3-Dimesitylimidazoliumchlorid wurden im Rahmen dieser Batch-Konzentrierung von der Membran weitestgehend zurückgehalten und reicherten sich im Reaktor (Retentat) an. Nachdem das gesamte Reaktionsprodukt über die Nanofiltrationsanlage im Reaktor um den volumetrischen Konzentrierungsfaktor von ca. 10 angereichert war, wurde der Versuch beendet.

Die Nanofiltration wurde hinsichtlich Permeatfluss und Rückhalt für Palladium untersucht. Tabelle 1 zeigt die Ergebnisse der Versuche gemäß Beispiel 1.

**Tabelle 1: Ergebnisse Beispiel 1**

| Verfahrensschri tt | Strombezeichnung | Hauptkomponenten [g], Pd [ppm] | Pd,Permeat [ppm] Rückhalt Pd (R) |
|---|---|---|---|
| Telo, Start | Einsatzstoff | 331,4 g Methanol | |
| | | 511,2 g 1,3 Butadien | |
| | | 0 g 1-Methoxy-2,7-octadien | |
| | | 21,1 ppm Pd | |
| Telo, Ende (=Start NF) | Reaktionsprodukt zur Nanofiltration (zur NF) | 255,9 g Methanol | Pd, P 1,85 ppm |
| | | 42,9 g 1,3 Butadien | R, Pd 91% |
| | | 544,2 g 1-Methoxy-2,7-octadien | |
| | | 20,6 ppm Pd | |
| NF, Ende | Retentat Nanofiltration | 25,2 g Methanol | Pd, P 16,2 ppm |
| | | 41,9 g 1,3 Butadien | R, Pd 90 % |
| | | 54,6 g 1-Methoxy-2,7-octadien | |
| | | 160 ppm Pd | |

Die Nanofiltration zeigte bei spezifischen Permeatflussleistungen zwischen 15 und 20 [kg/m²h] gegenüber dem Pd einen nahezu konstanten Membranrückhalt von etwa 90 %. Die Bilanz über die gesamte Nanofiltration zeigte, dass 78,1 % der ursprünglich eingesetzten Palladiummasse im Retentat verblieben, während 21,9 % der Pd-Masse dem System über das Permeat entzogen wurden. Ein orientierender Versuch zur weiteren Pd-Rückhaltung über eine zweite NF-Stufe zur Nachbehandlung des Mischpermeates ergab, dass auch dort der Pd-Rückhalt an der Membran noch bei 85 % lag.

## Patentansprüche

1. Verfahren zur Abtrennung eines Metallkomplexkatalysators aus einem Reaktionsgemisch erhalten aus einer Telomerisation,
**dadurch gekennzeichnet,**
**dass** der Metallkomplexkatalysator an zumindest einer Membran abgetrennt wird, die für Moleküle mit einer Molmasse bis 1000 g/mol durchlässig sind, wobei im Reaktionsgemisch vorhandener freier Ligand, ausgewählt aus Organophosphor- oder Carben-Ligand, an der Membran abgetrennt und in die Telomerisation zurückgeführt wird, und wobei die Membran ausgewählt ist aus Membranen, die als trennaktive Schicht eine alkali- und lösemittelstabile Nanofiltrationspolymerschicht aus Polyimiden (PI), aromatischen Polyamiden (PA), Polyamidimiden (PAI), Polybenzimidazolen, Acrylonitril/ Glycidylmethacrylat (PANGMA), Polybenzimidazolonen, Polyacrylnitril (PAN), Polyarylethersulfonen, Polyestern, Polyetheretherketonen (PEEK), Polycarbonaten (PC), Polytetrafluorethylen, Polybenzimidazol (PBI), Polyvinylidenfluorid (PVDF), Polypropylen (PP), Polydimethylsiloxan (PDMS), aufweisen, oder deren trennaktive Schicht aus Polymeren mit intrinsischer Mikroporosität (PIM) aufgebaut ist, oder deren trennaktive Schicht über eine hydrophobierte keramische Membran aufgebaut ist.

2. Verfahren nach Anspruch 1,
**dadurch gekennzeichnet,**
**dass** zwei oder mehr Membranmodule eingesetzt werden.

3. Verfahren nach einem der Ansprüche 1 oder 2,
**dadurch gekennzeichnet,**
**dass** die Abtrennung als druckgetriebener Prozess erfolgt.

4. Verfahren nach zumindest einem der Ansprüche 1 bis 3,
**dadurch gekennzeichnet,**
**dass** die Membranabtrennung so durchgeführt wird, dass ein Druckunterschied von Retentat- zu Permeatseite von mindestens 0,5 MPa vorliegt.

5. Verfahren nach zumindest einem der Ansprüche 1 bis 4,
**dadurch gekennzeichnet,**
**dass** das Verfahren mit einer Überströmgeschwindigkeit an der Membran von 0,1 bis 15 m/sec. durchgeführt wird.

6. Verfahren nach zumindest einem der Ansprüche 1 bis 5,
**dadurch gekennzeichnet,**
**dass** das Volumenstromverhältnis des auf die Membran im Querstrom geführten Stroms zu Permeatstrom von 100 bis 10000 zu 1 beträgt.

7. Verfahren nach zumindest einem der Ansprüche 1 bis 6,
**dadurch gekennzeichnet,**
**dass** der abgetrennte Metallkomplexkatalysator in die Telomerisation zurückgeführt wird.

8. Verfahren nach zumindest einem der Ansprüche 1 bis 7,
**dadurch gekennzeichnet,**
**dass** das Molekular-Volumenverhältnis des Organophosphor- oder Carben-Liganden zu Telomerisationsprodukt ≥ 1,5 beträgt.

9. Verfahren nach zumindest einem der Ansprüche 1 bis 8,
**dadurch gekennzeichnet,**
**dass** das Telomerisationsgemisch mit einem Druck und/oder einer Temperatur von 0 bis 30 % kleiner als unter den Reaktionsbedingungen der Telomerisation auf die Membran gefahren wird.

10. Verfahren nach zumindest einem der Ansprüche 1 bis 9,
**dadurch gekennzeichnet,**
**dass** als Reaktionsgemisch aus einer Telomerisation ein Telomerisationsgemisch eingesetzt wird, welches durch Telomerisation von nicht cyclischen Olefinen mit mindestens zwei konjugierten Doppelbindungen mit mindestens einem Nucleophil unter Verwendung eines Katalysators enthaltend ein Metall der Gruppe 8, 9 oder 10 des Periodensystems der Elemente, erhalten wird.

11. Verfahren nach zumindest einem der Ansprüche 1 bis 10,
**dadurch gekennzeichnet,**
**dass** ein Telomerisationsgemisch eingesetzt wird, in dem ein Palladiumcarbenkomplex als Metall-Komplexkatalysator vorhanden ist.

12. Verfahren nach Anspruch 11,
**dadurch gekennzeichnet,**
**dass** in der Telomerisation das Verhältnis von Carben- oder Organophosphor-Ligand zu Metall [Mol/Mol] von 0,01 zu 1 bis 250 zu 1 beträgt.

13. Verfahren nach zumindest einem der Ansprüche 1 bis 11,
**dadurch gekennzeichnet,**
**dass** in der Telomerisation als Nucleophil (VII) Verbindungen der allgemeinen Formeln R1a-O-H (VIIa) oder (R1a)(R1b)N-H (VIIb) oder R1a-COOH (VIIc) worin R1a und R1b unabhängig voneinander ausgewählt sind aus Wasserstoff, einer linearen, verzweigten oder cyclischen C1 bis C22-Alkylgruppe, einer Alkenylgruppe, einer Alkinylgruppe, einer C5 bis C18 Arylgruppe oder einer -CO-Alkyl-(C1-C8)-Gruppe oder einer -CO-Aryl-(C5-C10)-Gruppe, wobei diese Gruppen Substituenten ausgewählt aus der Gruppe -CN, -COOH, -COO-Alkyl-(C1-C8), -CO-Alkyl-(C1-C8), - Aryl-(C5-C10), -COO-Aryl-(C6-C10), -CO-Aryl-(C6-C10), -O-Alkyl-(C1-C8), -O-CO-Alkyl-(C1-C8), -N-Alkyl2-(C1-C8), -CHO, SO3H, -NH2, -F, -Cl, -OH, -CF3, -NO2 enthalten können, und wobei die Reste R1a und R1b über kovalente Bindungen miteinander verknüpft sein können, eingesetzt werden.

14. Verfahren nach zumindest einem der Ansprüche 1 bis 13,
**dadurch gekennzeichnet,**
**dass** die Abtrennung bei einer Temperatur von 80 bis 100 °C durchgeführt wird.

## Claims

1. Process for separating a metal complex catalyst from a reaction mixture obtained from a telomerization,
**characterized in that**
the metal complex catalyst is separated off at at least one membrane which is/are permeable to molecules having a molar mass of up to 1000 g/mol, where free ligand selected from among organophosphorus or carbene ligand which is present in the reaction mixture is separated off at the membrane and recirculated to the telomerization and the membrane is selected from among membranes which comprise, as separation-active layer, an alkali-and solvent-stable nanofiltration polymer layer of polyimides (PI), aromatic polyamides (PA), polyamidimides (PAI), polybenzimidazoles, acrylonitrileglycidyl methacrylate (PANGMA), polybenzimidazolones, polyacrylonitrile (PAN), polyaryl ether sulfones, polyesters, polyether ether ketones (PEEK), polycarbonates (PC), polytetrafluoroethylene, polybenzimidazole (PBI), polyvinylidene fluoride (PVDF), polypropylene (PP), polydimethylsiloxane (PDMS) or whose separation-active layer is made up of polymers having intrinsic microporosity (PIM) or whose separation-active layer is built up over a hydrophobicized ceramic membrane.

2. Process according to Claim 1,
**characterized in that**
two or more membrane modules are used.

3. Process according to either of Claims 1 and 2,
**characterized in that**
the separation is carried out as a pressure-driven process.

4. Process according to at least one of Claims 1 to 3,
**characterized in that**
the membrane separation is carried out so that there is a pressure difference from the retentate side to the permeate side of at least 0.5 MPa.

5. Process according to at least one of Claims 1 to 4,
**characterized in that**
the process is carried out at a flow velocity over the membrane of from 0.1 to 15 m/sec.

6. Process according to at least one of Claims 1 to 5,
**characterized in that**
the volume flow ratio of the stream fed to the membrane in cross-current to permeate stream is 100 - 10 000:1.

7. Process according to at least one of Claims 1 to 6,
**characterized in that**
the metal complex catalyst which is separated off is recirculated to the telomerization.

8. Process according to at least one of Claims 1 to 7,
**characterized in that**
the molecular volume ratio of the organophosphorus or arbine ligand to the telomerization product is ≥1. 5.

9. Process according to at least one of Claims 1 to 8,
**characterized in that**
the telomerization mixture is fed to the membrane at a pressure and/or a temperature which are/is from 0 to 30% lower than under the reaction conditions of the telomerization.

10. Process according to at least one of Claims 1 to 9,
**characterized in that**
a telomerization mixture which is obtained by telomerization of acyclic olefins having at least two conjugated double bonds with at least one nucleophile using a catalyst containing a metal of group 8, 9 or 10 of the Periodic Table of the Elements is used as reaction mixture from a telomerization.

11. Process according to at least one of Claims 1 to 10,
**characterized in that**
a telomerization mixture in which a palladium-carbene complex is present as metal complex catalyst is used.

12. Process according to Claim 11,
**characterized in that**
the ratio of _arbine or organophosphorus ligand to metal [mol/mol] in the telomerization is from 0.01:1 to 250:1.

13. Process according to at least one of Claims 1 to 11,
**characterized in that**
compounds of the general formula
R^{1a}- O-H (VIIa) or (R^{1a}) (R^{1b}) N-H (VIIb) or R^{1a}-COOH (VIIc)
where R^{1a} and R^{1b} are selected independently from among hydrogen, a linear, branched or cyclic C₁-C₂₂-alkyl group, an alkenyl group, an alkynyl group, a C₅-C₁₈-aryl group or a -CO-alkyl-(C₁-C₈) group or a -CO-aryl-(C₅-C₁₀) group, these groups being able to contain substituents selected from the group consisting of -CN, -COOH, -COO-alkyl-(C₁-C₈), -CO-alkyl- (C₁-C₈), -aryl- (C₅-C₁₀), -COO-aryl- (C₆-C₁₀), -CO-aryl-(C₆-C₁₀), -O-alkyl-(C₁-C₈), -O-CO-alkyl-(C₁-C₈) , -N-alkyl₂-(C₁-C₈), -CHO, -SO₃H, -NH₂, -F, -Cl, -OH, -CF₃, -NO₂, and the radicals R^{1a} and R^{1b} being able to be joined to one another via covalent bonds, are used as nucleophile (VII) in the telomerization.

14. Process according to at least one of Claims 1 to 13,
**characterized in that**
the separation is carried out at a temperature of from 80 to 100°C.

## Revendications

1. Procédé de séparation d'un catalyseur de complexe métallique d'un mélange réactionnel obtenu par une télomérisation,
**caractérisé en ce que**
le catalyseur de complexe métallique est séparé sur au moins une membrane qui est perméable aux molécules ayant une masse moléculaire jusqu'à 1 000 g/mol, un ligand libre présent dans le mélange réactionnel, choisi parmi un ligand organophosphore ou carbène, étant séparé sur la membrane et recyclé dans la télomérisation, et la membrane étant choisie parmi les membranes qui comprennent en tant que couche à action de séparation une couche polymère de nanofiltration stable en milieux alcalins et en solvants à base de polyimides (PI), polyamides aromatiques (PA), polyamide-imides (PAI), polybenzimidazoles, acrylonitrile/méthacrylate de glycidyle (PANGMA), polybenzimidazolones, polyacrylonitrile (PAN), polyaryléthersulfones, polyesters, polyétheréthercétones (PEEK), polycarbonates (PC), polytétrafluoroéthylène, polybenzimidazole (PBI), polyfluorure de vinylidène (PVDF), polypropylène (PP), polydiméthylsiloxane (PDMS), ou dont la couche à action de séparation est formée par des polymères à microporosité intrinsèque (PIM), ou dont la couche à action de séparation est formé par une membrane céramique hydrophobée.

2. Procédé selon la revendication 1, **caractérisé en ce que** deux modules de membrane ou plus sont utilisés.

3. Procédé selon la revendication 1 ou 2, **caractérisé en ce que** la séparation a lieu sous la forme d'un procédé actionné par pression.

4. Procédé selon au moins l'une quelconque des revendications 1 à 3, **caractérisé en ce que** la séparation sur membrane est réalisée de manière à ce qu'une différence de pression entre le côté du rétentat et le côté du perméat d'au moins 0,5 MPa soit présente.

5. Procédé selon au moins l'une quelconque des revendications 1 à 4, **caractérisé en ce que** le procédé est réalisé à une vitesse d'écoulement sur la membrane de 0,1 à 15 m/s.

6. Procédé selon au moins l'une quelconque des revendications 1 à 5, **caractérisé en ce que** le rapport entre les débits volumiques du courant conduit sur la membrane en courant transversal et du courant de perméat est de 100 à 10 000 sur 1.

7. Procédé selon au moins l'une quelconque des revendications 1 à 6, **caractérisé en ce que** le catalyseur de complexe métallique séparé est recyclé dans la télomérisation.

8. Procédé selon au moins l'une quelconque des revendications 1 à 7, **caractérisé en ce que** le rapport entre les volumes moléculaires du ligand organophosphore ou carbène et du produit de télomérisation est ≥ 1,5.

9. Procédé selon au moins l'une quelconque des revendications 1 à 8, **caractérisé en ce que** le mélange de télomérisation est conduit sur la membrane à une pression et/ou une température inférieures de 0 à 30 % par rapport aux conditions de réaction de la télomérisation.

10. Procédé selon au moins l'une quelconque des revendications 1 à 9, **caractérisé en ce qu'**un mélange de télomérisation qui est obtenu par télomérisation d'oléfines non cycliques contenant au moins deux doubles liaisons conjuguées avec au moins un nucléophile en utilisant un catalyseur qui contient un métal du groupe 8, 9 ou 10 du tableau périodique des éléments est utilisé en tant que mélange réactionnel issu d'une télomérisation.

11. Procédé selon au moins l'une quelconque des revendications 1 à 10, **caractérisé en ce qu'**un mélange de télomérisation dans lequel un complexe palladium-carbène est présent en tant que catalyseur de complexe métallique est utilisé.

12. Procédé selon la revendication 11, **caractérisé en ce que** le rapport entre le ligand carbène ou organophosphore et le métal [mol/mol] dans la télomérisation est de 0,01 sur 1 à 250 sur 1.

13. Procédé selon au moins l'une quelconque des revendications 1 à 11, **caractérisé en ce que** des composés de formules générales R^{1a}-O-H (VIIa) ou (R^{1a}) (R^{1b})N-H (VIIb) ou R^{1a}-COOH (VIIc) , R^{1a} et R^{1b} étant choisis indépendamment l'un de l'autre parmi l'hydrogène, un groupe alkyle en C₁ à C₂₂ linéaire, ramifié ou cyclique, un groupe alcényle, un groupe alcynyle, un groupe aryle en C₅ à C₁₈ ou un groupe -CO-alkyle-(C₁-C₈) ou un groupe -CO-aryle- (C₅-C₁₀), ces groupes pouvant contenir des substituants choisis dans le groupe constitué par -CN, -COOH, -COO-alkyle-(C₁-C₈), -CO-alkyle-(C₁-C₈), -aryle-(C₅-C₁₀), -COO-aryle-(C₆-C₁₀), -CO-aryle- (C₆-C₁₀), -O-alkyle- (C₁-C₈), -O-CO-alkyle-(C₁-C₈) , -N-alkyle₂-(C₁-C₈), -CHO, SO₃H, -NH₂, -F, -Cl, -OH, -CF₃, -NO₂, et les radicaux R^{1a} et R^{1b} pouvant être reliés l'un à l'autre par des liaisons covalentes, sont utilisés en tant que nucléophile (VII) dans la télomérisation.

14. Procédé selon au moins l'une quelconque des revendications 1 à 13, **caractérisé en ce que** la séparation est réalisée à une température de 80 à 100 °C.
